# EUROPEAN PATENT SPECIFICATION

(11) **EP 2 872 898 B1**
(45) Date of publication and mention of the grant of the patent: **07.12.2016**
(21) Application number: 13759843.9
(22) Date of filing: 15.07.2013
(51) Int. Cl.: C12Q 1/37, G01N 33/487, G01N 33/58, G01N 33/68

(54) **SINGLE MOLECULE PROTEIN SEQUENCING**
EINZELMOLEKÜL-PROTEINSEQUENZIERUNG
SÉQUENÇAGE D'UNE PROTÉINE À MOLÉCULE UNIQUE

(30) Priority: 16.07.2012 NL 2009191
(43) Date of publication of application: 20.05.2015
(73) Proprietor: Technische Universiteit Delft, 2628 CN Delft (NL)
(72) Inventor: JOO, Chirlmin, 2600 AA Delft (NL); DEKKER, Cornelis, 2600 AA Delft (NL); VAN GINKEL, Hendrika Geertruida Theodora Maria, 2600 AA Delft (NL); MEYER, Anne Sara, 2600 AA Delft (NL)
(74) Representative: Ellens, Andries
(86) International application number: PCT/NL2013/050537
(87) International publication number: WO 2014/014347

(56) References cited:
- WO-A1-2005/052591
- WO-A2-02/055189
- DE-A1-102006 049 682
- US-A1- 2003 207 326
- US-A1- 2004 038 307
- US-A1- 2004 197 793
- US-A1- 2006 014 212
- RODRIGOA MAILLARD ET AL: "ClpX(P) Generates Mechanical Force to Unfold and Translocate Its Protein Substrates", CELL, CELL PRESS, US, vol. 145, no. 3, 14 April 2011 (2011-04-14), pages 459-469, XP028201068, ISSN: 0092-8674, DOI: 10.1016/J.CELL.2011.04.010 [retrieved on 2011-04-19]
- MARIE-EVE AUBIN-TAM ET AL: "Single-Molecule Protein Unfolding and Translocation by an ATP-Fueled Proteolytic Machine", CELL, CELL PRESS, US, vol. 145, no. 2, 11 March 2011 (2011-03-11), pages 257-267, XP028194592, ISSN: 0092-8674, DOI: 10.1016/J.CELL.2011.03.036 [retrieved on 2011-03-24]

## Description

### FIELD OF THE INVENTION

The invention relates to a (single molecule) method for determining the type of a protein (by sequencing), as well as to a device that can be used for such method.

### BACKGROUND OF THE INVENTION

Methods for single molecule protein analysis are known in the art. WO2010065531, for instance, describes that such methods can be used for discovery of new biomarkers, quantitation, and high throughput screening. It is indicated that surface bound peptides are able to be directly sequenced using a modified Edman degradation followed by detection, e.g., labeled antibody detection. High throughput screening is enabled using pools of molecules (e.g., labeled antibodies) to identify and quantitate individual protein analytes in a biological sample.

Further, WO2010144151 describes compositions, methods, and systems for performing single-molecule, real-time analysis of analytical reactions in which protein synthesis is occurring. The ability to analyze such reactions provides an opportunity to study those reactions as well as to potentially identify factors and/or approaches for impacting such reactions, e.g., to either enhance, inhibit, or otherwise affect such reactions including, but not limited to, affecting the reaction rate, processivity, fidelity, duration, and the like. This document especially describes a method of determining a sequence of amino acids encoded by a target mRNA molecule, comprising: a) providing a reaction mixture comprising the target mRNA molecule, a ribosome complex comprising tMet-tRNA tMet in the P site, and a plurality of types of labeled aminoacyl-tRNAs free in solution, wherein the ribosome and/or the target mRNA molecule is immobilized upon a support such that an observation volume contains no more than one ribosome and/or mRNA molecule, and further wherein the ribosome complex does not comprise a detectable label or a quenching group; b) initiating a processive translation of the mRNA molecule by the ribosome complex; c) during said processive translation, sequentially and optically detecting association of the ribosome complex with at least a first labeled aminoacyl-tRNA and a second labeled aminoacyl-tRNA, where said association results in an incorporation of a first amino acid from the first labeled aminoacyl-tRNA and a second amino acid from the second labeled aminoacyl-tRNA into a nascent polypeptide chain; and d) identifying the first amino acid and the second amino acid, thereby determining a sequence of amino acids encoded by the target mRNA molecule.

### SUMMARY OF THE INVENTION

Proteins are the basis of life as they are the working machineries in all forms of life. To understand biological phenomena, it is required to have comprehensive knowledge of the proteins involved. Protein sequencing, determining the amino acid sequence of a protein, is used to obtain a profile of protein populations, from cell lines to cell tissues to individual organisms. Since the first protein sequencing of insulin in the 1950s, sequencing technology has steadily evolved to open the era of proteomics, the comprehensive mapping of cellular proteins.

Modern protein sequencing is mainly based on mass spectrometry techniques (ESI, MALDI, etc.). Each has its own advantages and disadvantages, but all of them share the same limitations. First, they can analyze only protein fragments (about 10-20 amino acids). When full-length proteins (typically several hundred amino acids long) are examined, a computational complication prohibits accurate sequence prediction. Second, they often fail to recognize minor species embedded among other dominant species since sequence prediction is made through analysis of complex spectral peaks. As many cellular proteins exist in low abundance, this makes it difficult to obtain large-scale proteomic information.

In DNA sequencing, similar challenges are faced, but they are overcome when DNA samples are amplified until a high signal-to-noise ratio is achieved. Unlike DNA, there is no natural machinery that can amplify proteins. Here we aim to develop an entirely novel method that can quantify cellular proteins with accuracy as high as for large-scale techniques, and using sample amounts as small as a single cell.

Hence, it is an aspect of the invention to provide an alternative (single molecule) (sequencing) method for determining the type of a protein and/or an alternative device for determining the type of a protein, especially suitable for use in such alternative (single molecule) method, which method and/or device preferably further at least partly obviate one or more of above-described limitations.

Therefore, a novel sequencing method using a (in an embodiment) single-molecule fluorescence technique is proposed, as defined in the accompanying claims. This new approach will explore proteins, molecule by molecule, not just take their average; thus, it may cover entire proteins despite the complex nature and the wide dynamic range of cellular proteins. Unlike mass spectrometry-based sequencing, this approach will read the sequence of full-length proteins, which will make the sequencing prediction less error-prone. Single-molecule detection is so sensitive that this approach may require only a small amount of sample (no more than 1 fmol) for the analysis of cellular proteins. This will create the opportunity for single-cell analysis. These advantages contrast with the limitations of mass spectrometry, which typically requires 10³-10⁵ times more proteins for analysis.

The new method can be marked by three novel concepts: (1) fingerprinting, (2) enzyme-based manipulation, and (3) real-time sequencing. Since our analysis suggests that a protein sequence can be predicted with reading (already) only two types of amino acids (see about the prediction power below), proteins can be identified by probing two different amino acids only, such as cysteine and lysine residues only. To control the sequencing process with nanometer accuracy, a chaperone protein (e.g. ClpXP) can be applied, though other suitable molecular transporter machines can be applied as well. Using (in an embodiment) single-molecule fluorescence microscopy, we will watch individual sequencing substrates being probed by e.g. single ClpXP proteins in real time. Hence, the novel technique is especially suitable for sequencing of proteins having at least 300 amino acids, even more especially at least 600 amino acids. Hence, even with as few as about 300 amino acids sequencing may be performed (although in some cases an even lower number may be possible; see also below).

In another embodiment (see below), a nanopore technique is applied.

The invention provides a method for analysing a protein in a liquid (comprising the protein), especially for determining the type of a protein in a liquid comprising the protein, the method comprising (a) functionalizing a protein with amino acid labels, especially at least 2, such as 2-8, especially 2-4, like only 2, types of, amino acid labels, which are selective for especially at least 2, such as 2-8, especially 2-4, like only 2, types of, predefined protein amino acids, especially only two amino acid labels selective for only two amino acids, especially the C and K amino acids, (b) guiding in the liquid phase the functionalized protein with an immobilized ATP dependent protease based molecular transporter machine through a detection area of a detector, configured to detect a signal as function of the labels of the labelled amino acids (when the guiding the functionalized protein is guided through a detection area of the a detector (with the with the immobilized ATP dependent protease based molecular transporter machine)); (c) determining from the detected signal a sequence of the predefined amino acids; and optionally (d) comparing the sequence of the predefined amino acids with the occurrence of such sequence in a database of proteins and determining the type of protein in the liquid. The term "amino acid label" refers to a label for an amino acid. Herein, instead of "amino acid label" also the term "label" is applied. These labels are especially dyes, see also below.

Further, the invention provides a device for determining the type of protein in a liquid, the device comprising (a) an immobilized ATP dependent protease based molecular transporter machine configured to guide a protein that is functionalized with labels (i.e. some (predetermined) amino acids are functionalized with labels) through a detection area of a detector, (b) said detector, configured to detect a signal as function of the labels of the labelled amino acids, (c) a processor unit, configured to identify from the detector signal a sequence of amino acids of the functionalized protein, wherein (d) the processor unit is optionally further configured to compare the identified sequence of amino acids with the occurrence of such sequence in a database of proteins and to (based on the comparison) identify the type of protein, as further defined in the accompanying claims. Hence, the device may comprise an immobilized ATP dependent protease based molecular transporter machine configured to guide a protein that is functionalized with amino acid labels, which are selective for (especially 2-4 types of) predefined protein amino acids, through a detection area of a detector.

This revolutionary single-molecule approach will provide a novel deep sequencing tool for protein analysis. Expected are diverse applications in biology, biotechnology, and medical sciences. When this technique is developed to a table-top tool in the future, we anticipate that medical researchers will be able to elucidate protein expression profiles by tracking variations among individuals, among different tissues, and under different environmental conditions. This novel sequencing technique can change the paradigm of proteomics and may become a universal diagnostic tool.

An immediate challenge for single-molecule protein sequencing is that proteins are composed of 20 different amino acids. Unlike DNA sequencing, which distinguishes only four different nucleotides (A, G, C, and T) and requires only four fluorophores, full protein sequencing demands 20 fluorescent tags. However, it is practically impossible to find 20 fluorophores whose spectra do not overlap with one another.

We will thereby bring about a new concept, 'protein finger-printing,' by approaching protein sequencing from a very different angle than DNA sequencing. Let us first ask the question of how much information we need for protein identification. DNA sequencing requires accurate readout of every nucleotide; otherwise, the information obtained is meaningless due to deletions, insertions, and mutations. Protein sequencing, on the other hand, does not require authentic reading of all the amino acids. Rather, with reference to public genomic and proteomic databases, the sequencing can be reduced into a problem of identifying proteins out of a pool of protein populations (for example, ∼20,000 species in human cells) in each organism.

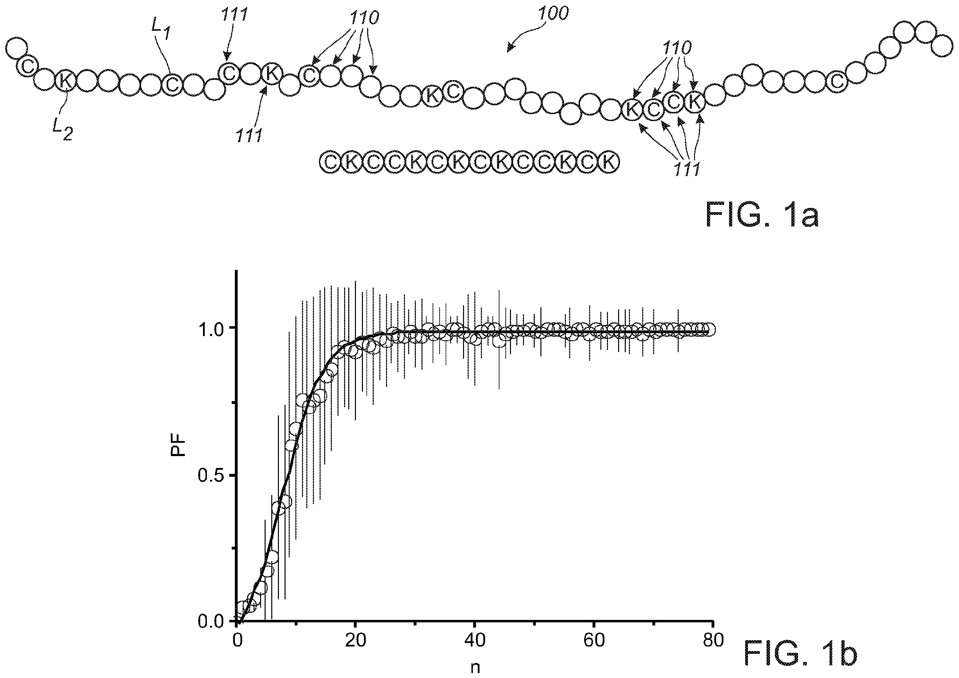

Let's assume that we only read two types of amino acids. With this two-bit information, how many amino acids should we read in series to identify a protein? A mathematical estimate, 2¹⁴ < 20,000 < 2¹⁵, suggests that we only need to read 15 amino acids or more. To convert the 20-bit information of protein peptides into 2-bit, we will target the two highly nucleophilic amino acids that can be labeled both efficiently and specifically - lysine (Lys, K) and cysteine (Cys, C). As displayed in Fig. 1a (see also below), a read-out of K and C amino acids will be registered as `...CKCCKCKCKCCKCK...,' and this data will be used to predict the protein identity. K is observed 1 out of 20 amino acids on average; and C, 1 out of 40. A typical protein is ∼400 amino acids long, and so contains 30 residues of K and C on average. This number is well above the minimum number, 15 amino acids, which is (in general) required for identification with reference to a protein database (see however further also below).

Whereas the prediction above is based on simple mathematical estimation, an estimation on the practical prediction power of fingerprinting was carried out using the human protein database Uniprot (www.uniprot.org). A computational fingerprinting analysis using a complete and reviewed proteome of the human organism (No.9606) was carried out. All the positional information from the database was discarded and each complete protein sequence was reduced to the sequences of the C and K amino acids only (a C-K database). Given a simulated sequence, our program compared it with all the sequences in the C-K database and suggested the best match(es). The prediction fidelity is defined as the inverse of the number of the best matches. For these computational analyses, we adopted both a point-point matching algorithm (which is based on correlation analysis) and the Smith-Waterman algorithm (Smith, Temple F.; and Waterman, Michael S. (1981). "Identification of Common Molecular Subsequences". Journal of Molecular Biology 147: 195-197).

Our analysis demonstrates that the prediction power reaches a satisfactory level when about 17 amino acids or longer are read (Fig. 1b). This number is slightly larger than the 15 amino acids we estimated, but much smaller than the average number of K and C residues, 30, per protein. Hence, the method especially involves detecting the presence of at least 15, especially at least 17, of the predefined protein amino acids in the protein to be identified. This may especially apply to human proteins. For non-human proteins (and/or for non-animal proteins), it may be that less than at least 15 amino acids can be labeled, and still obtain a good predictability. Further, it is noted that in case the technique is used to determine the type of protein out of a limited number of predetermined proteins, also the number of at least 15 amino acids may be lower.

Note that the phrase "detecting the presence of at least 15, especially at least 17, of the predefined protein amino acids in the protein" does not exclude the method to further sequence and measure more than only 15, or only 17 of the predetermined proteins. It is not excluded to measure more than e.g. (in total) 15 amino acids K and C, such as over 20, like over 40 amino acids.

Further, based on the above principle, it is suggested to label 2-8 different amino acids, especially only 2-4, such as even only 2 different amino acids, such as especially Lys (K) and Cys (C). Herein, the term "protein amino acid" and similar terms are used, to indicate that the invention relates to amino acids that are known to be available in proteins. Instead of this term, also the short term "amino acids" is herein applied. The term "protein" especially relates to naturally occurring proteins, such as human, animal or plant proteins.

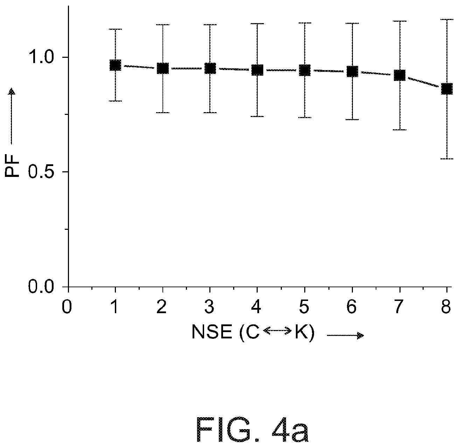

We note that intrinsic measurement errors and incomplete fluorophore labeling may interfere with the prediction, as they will lead to apparent deletions, insertions, or swappings of Lys and Cys signals. Our error analysis indicates that identification of human proteins is tolerable to ∼10% of error in fluorescence labeling and measurement. For example, our analysis (Figure 4a) shows that the predication fidelity (PF) does not drop significantly even when there are a number of swapping errors (NSE) present (i.e. the order of C and K is swapped during scanning) given a 40-long CK sequence. We performed this error analysis using both a point-point matching algorithm and the Smith-Waterman algorithm.

To read C and K residues of a protein, we need a specific nano-apparatus that unfolds the protein from a complicated three-dimensional to a linear one-dimensional conformation and scans it with nanometer accuracy. We propose an enzyme-based real-time approach, using a tool to read quickly (such as 0.1-60 amino acids/sec) without any chemical reactions being involved. In this perspective, we aim to use a naturally existing protein that translocates along a protein substrate with tight interaction. The real-time DNA sequencing uses an enzyme (DNA polymerase) that binds to DNA and scans the DNA strand, which action naturally reports on the DNA sequence.

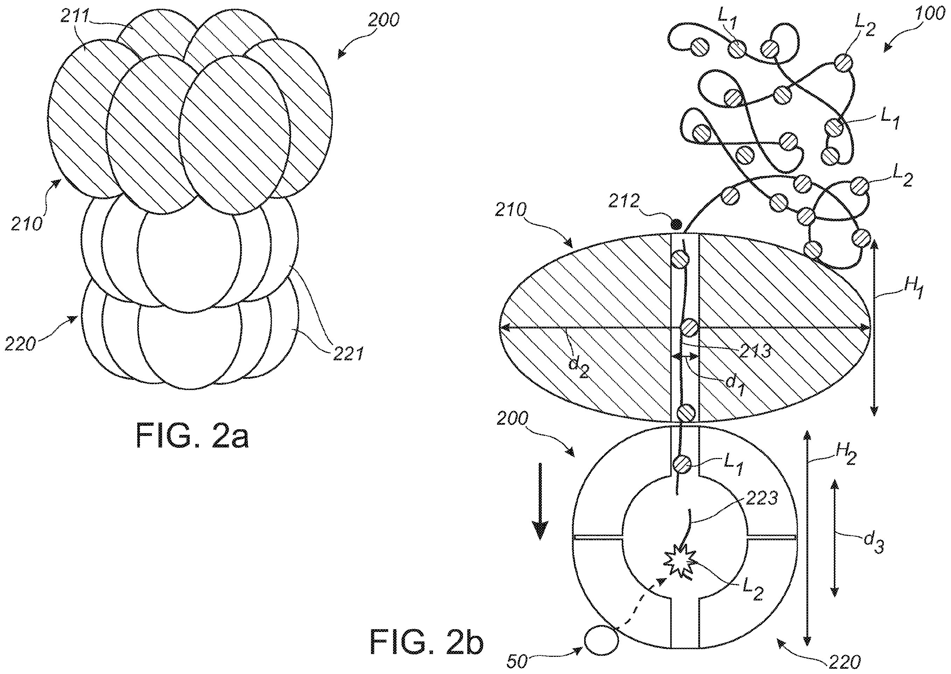

ClpXP (a protein complex of ClpX and ClpP) is a chaperone complex that unfolds a protein substrate and translocates along it (Fig. 2a). It is a processive and fast enzyme that keeps in close contact with its substrate. A single ClpXP can translocate along concatenated monomers (eight 20kD monomers, >1000 amino acids long). ClpXP can translocate as fast as 60 amino acids per second (Maillard, R.A., Chistol, G., Sen, M., Righini, M., Tan, J., Kaiser, C.M., Hodges, C., Martin, A., and Bustamante, C. (2011). ClpX(P) generates mechanical force to unfold and translocate its protein substrates. Cell 145, 459-469). ClpX has a nanometer-wide channel, and ClpP has nanometer-wide pores, the dimensions of which provide the capability of manipulating a sequencing substrate with nanometer accuracy. Biochemical properties of ClpXP such as substrate specificity and resistance to protein modifications are well-characterized. ClpXP can translocate and degrade modified substrates such as chemically denatured substrates, dye-labeled substrates, and chemically cross-linked proteins. ClpXP seems highly promiscuous in substrate processing. It has been found that modification of sequencing substrates will not interfere with ClpXP activity. Single-molecule sequencing may also require modification of ClpP or ClpX. The mutation and dye-labeling of ClpP and ClpX appears to be feasible.

However, other ATP dependent proteases may be applied as well. In an embodiment, the molecular transporter machine is a molecular transporter machine selected from the group consisting of i.a. a ClpXP, a ClpAP, a ClpCP, a ClpEP, a ClpYQ (an HslUV), a ClpB, a Lon, an FtsH, an archeal PAN and a proteasome based molecular transporter machine (see also Kirstein, J., Mollière, N., Dougan, D.A., and Turgay, K., Adapting the machine: adaptor proteins for Hsp100/Clp and AAA+ proteases, Nature Reviews - Biology, 7, August 2009, 589-599), especially a ClpXP based molecular transporter machine. However, also other (ATP dependent protease) molecular transporter machines may be applied. These ATP dependent proteases share a common basic structure and mechanism of action, in which the AAA+ enzyme may select protein substrates and translocates along them processively, while the protease may degrade the protein substrates. In a specific embodiment, ClpXP is applied. In yet another embodiment, ClpAP is applied.

Using ClpXP as a scanning probe, fluorescence techniques can be applied as follows. We first need to label sequencing substrates with dyes. We choose, in a specific embodiment, highly nucleophilic amino acids, lysine (K) and cysteine (C). The amine group of K will be conjugated with NHS-ester dye and the thiol group of C with maleimide dye. The two reactions are orthogonal to each other, which prevents cross-labeling. These chemical reactions occur so efficiently, reaching ∼100% yield under a general reaction condition (a micro molar concentration of reactive dyes and several hours of incubation), that several labeling kits are commercially available. This makes the labeling procedure time- and cost-effective. To ensure complete labeling of C and K residues, we will expose internal amino acids through protein denaturation. Instead of or in addition to lysine and cysteine, also serine, threonine, tyrosine, and post-translationally modified amino acids may be chosen. Hence, especially two or more of lysine, cysteine, serine, threonine, tyrosine, and post-translationally modified amino acids may be chosen to be labeled, especially two or more of lysine, cysteine, serine, threonine, and tyrosine. In an embodiment, only cysteine and lysine are labeled.

Among several denaturation methods, SDS (sodium dodecyl sulfate)-mediated and heat-induced denaturation can be employed, which is effective in disrupting secondary and tertiary protein structures. In order to break disulfide bridges, strong reducing reagents (such as β-mercaptoethanol), under this harsh denaturing condition, can be used. In the subsequent dye-labeling step, reducing reagents will preferably be removed since they may interfere with (cysteine) labeling. This removal can e.g. be carried out with a general protein precipitation procedure (with acetone, ammonium sulfate, or polyethylenimine). After dye labeling a buffer exchange (through dialysis or a size exclusion column) or an additional round of protein precipitation to eliminate excess dyes can be carried out. These purification steps may especially guarantee that enzyme-mediated sequencing reactions run in biologically optimal conditions.

As a result of the labeling, dyes will be interspersed along a sequencing substrate over in general a few nanometers of distance. To resolve them in order, we need a nanometer-resolution imaging method. Here we introduce a nanometer ruler, FRET (fluorescence resonance energy transfer between donor and acceptor fluorophores) (Roy, R., Hohng, S., and Ha, T. (2008). A practical guide to single-molecule FRET. Nat Methods 5, 507-516). Below, we also introduce a nanopore filter, than can be used to resolve the order of the labels. With K and C residues labeled with two different colors of acceptor dyes respectively (Cy5 and Cy7), the acceptor molecules can be probed by scanning with a Cy3 donor molecule and measuring FRET of Cy5 and Cy7 fluorescence signals with Cy3. Other acceptor dyes such as Cy3.5 and Cy5.5 (for other amino acids) may be probed in an analogous way. Hence, the labels may comprise an organic fluorophore selected from e.g. one or more of the Cyanine family, the Alexa family, the Atto family, the Dy family, and the Rhodamine family, though other fluorophores are not excluded.

As illustrated in Fig. 2b, ClpX (a hexameric ring, shown as an ellipse) has an entry port at the top, and ClpP (a dimer of heptameric rings) has a putative exit port at the bottom. These two proteins, tightly bound, are about 16 nm in height and about 9-15 nm in width. When a sequencing substrate docks onto the ClpX entry port via its tag, the substrate is translocated into the narrow channel (1 nm wide) of ClpX and becomes unfolded. The unfolded protein is handed over to its partner protein, the ClpP protease, which then cleaves the translocated peptide into small fragments.

ClpXP recognizes substrates displaying a certain specific tag only. A well-known tag, λO ((TNTAKILNFGR) SEQ ID NO:1) (Farell, C.M., Baker, T.A, and Sauer, R.T., Altered specificity of a AAA+ protease, Molecular Cell 25,161-166, January 12, 2007,161-166-), can be ligated at the N-teiminus of sequencing substrates. EDC chemistry can be used to conjugate the C-terminus (carboxyl group) of the synthetic O peptide with the N-terminus (amino group) of the sequencing substrates. Since the amino group of Lys is also targeted by EDC chemistry, this ligation requires careful considerations. First, as the pK value of the alpha amino group (pKa=8.9) is lower than that of the epsilon amino group of Lys (pKa=10.5), the EDC chemistry can be carried out at a pH of 6.5 - 8.5. Second, the ligation reaction can be carried out after the Lys labeling at pH 9.0-11.0, to minimize any non-specific ligation. Instead of the N-terminal λO (TNTAKILNFGR) tag, also a C-terminal tag ssrA ((AANDENYALAA) SEQ ID NO:2) can be applied, or any other N- or C-terminal tag (see also Flynn J.M., Neher, S.B., Kim, Y., Sauer, R.T., Baker, A.T., Proteomic Discovery of Cellular Substrates of the ClpXP Protease Reveals Five Classes of ClpX-Recognition Signals, Molecular Cell, Viol.11, March, 2003, 671-683). Hence, in an embodiment, the method further comprises taging the protein with a tag (or label) that is recognizable by the molecular transporter machine.

The radius of the ClpP chamber is ∼5 nm. This dimension is optimal in exploiting FRET sensitivity as the Cy3-Cy5 pair is most sensitive at 6 nm and the Cy3-Cy7 at 4 nm. If we place the donor (Cy3) at a ClpP chamber, we can use this optimal distance for measuring FRET between the donor (Cy3) and the acceptors (Cy5 and Cy7) of a peptide fragment. In addition, we may avoid in this way non-specific FRET between the donor and a pool of acceptors near the entry port since the distance between the entry port and the ClpP chamber is 7.5-12.5 nm, larger than regular FRET distances (which are in the order 4-6 nm). Alternatively, ClpX can be labeled with a Cy3 donor fluorophore. Further, Cy3, Cy5 and Cy7 refer to cyanine dyes, known in the art, such as e.g. described in Lee et al, 2010 (Lee, J., Lee, S., Ragunathan, K., Joo, C., Ha, T., and Hohng, S. (2010). Single-molecule four-color FRET. Angew. Chem Int. Ed. Engl. 49, 9922-9925). However, also other labels may be applied, selected from one or more of the Cyanine family, the Alexa family, the Atto family, the Dy family, and the Rhodamine family.

FRET from the donor will occur with any acceptor molecules within a ClpP chamber. Thereby, to obtain high-quality FRET time traces, it is essential to have as few dyes within a chamber at a time as possible. To achieve this, our sequencing technique utilizes the unique feature of ClpP. ClpP cleaves a protein into fragments and releases each fragment out of the chamber. Especially when slowing down the ClpX translocation speed enough (such as by decreasing the amount of ATP energy available) (Martin, A., Baker, T.A., Sauer, R.T., Protein unfolding by a AAA+ protease is dependent on AT-hydrolysis rates and substrate energy landscapes, Nature Structural & Molecular Biology, volume 15, no. 2, February 2008, 139-145; Shin, Y, Davids, J.H., Brau, R.R., Martin, A., Kenniston, J.A., Baker, Sauer, R.T., Lang, M.J., Single-molecule denaturation and degradation of proteins by the AAA+ ClpXP protease, PNAS, November 17, 2009, vol. 106, no. 46, 19340-19345), there will only be one cleavage reaction that occurs within the ClpP chamber at a time, which is followed by the diffusion-governed release of the single fragment. This controlled translocation scheme will keep the number of fragments within a chamber lower than one on average, which will enable interpreting a FRET time trace with minimal ambiguity. Relatedly, this assay will also guarantee that the order of the fragment release follows the same order of the original protein sequence, which minimizes any swapping errors in readout (C←→K).

Labeling of either ClpX or ClpP with donor and biotin may be carried out by conjugating the thiol group of Cysteine with maleimide-Cy3 dye and maleimide-biotin, respectively. Both ClpX and CIpP from *E. coli* have two Cysteine residues per monomer. Since both amino acids are not conserved across bacterial species, we can knock both of them out and introduce new Cysteine residues where Cy3 and biotin should be positioned. The crystal structures of the ClpX and CIpP protein will be utilized when assigning the position of the Cysteine point mutation.

A ClpP chamber is composed of 14 ClpP monomers, which raises two practical issues. First, due to this oligomeric structure, the chamber may be labeled with more than one donor molecule. Since we may only analyze acceptor signals, the multiple donor dyes will not interfere with our measurement. In fact, we may intentionally add more than one donor molecule to elongate the total observation time. Second, because of the point symmetry of the ClpP chamber, random positioning of a donor molecule may result in it being placed on an undesired side, i.e. on the interface between ClpX and ClpP rather than adjacent to the exit port. We can resolve this issue in an embodiment by generating an asymmetric ClpP chamber, in which one of the rings is composed of mutant ClpP that cannot interact with ClpX. We will dye-label only this mutant ClpP before assembling the chamber. The procedure as described by Maglica et al. (Maglica, Z., Kolygo, K., and Weber-Ban, E., Optimal efficiency of ClpAP and ClpXP chaperone-proteases is achieved by architectural symmetry, Structure 17, 2009, 508-516.) can be applied. Hence, especially the molecular transporter machine is a ClpXP based molecular transporter machine, wherein the ClpXP based molecular transporter machine comprises an asymmetric ClpP chamber of ClpP monomers and at least one mutant ClpP monomer, in which the at least one mutant ClpP monomer cannot dock to ClpX, and wherien this at least one mutant ClpP is fluorescent donor labelled (before assembling the chamber). It is noted that, in case ClpX is labeled with a donor fluorophore, it is not necessary to create an asymmetric ClpP protein. Hence, in another embodiment the molecular transporter machine is a ClpXP based molecular transporter machine, wherein the ClpX is labelled with a donor fluorophore.

Therefore, more in general, the molecular transporter machine may comprise a donor, especially a fluorescent donor (donor fluorophore), which donor may especially be configured to probe an amino acid label. This may include a fluorescent donor labelled ClpXP or a fluorsecent donor labelled ClpAP, etc. ClPX or ClPA, etc. may be labelled. However, optionally in addition or alternatively, ClpP may be labelled with the fluorescent donor (donor fluorophore). Optionally two or more labels may be applied.

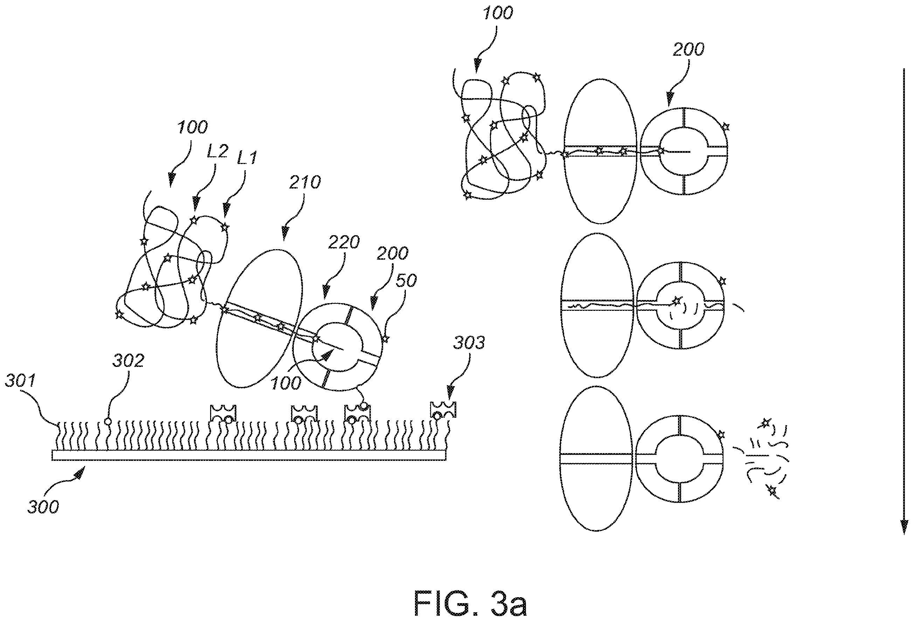

To observe sequencing events with single-molecule fluorescence, we may immobilize ClpXP proteins on a quartz surface and will image with TIRF (total internal reflection fluorescence) microscopy. The immobilization can be conducted by tagging ClpX or ClpP proteins with biotin and introducing them onto a streptavidin-layered quartz surface (Figs. 3a-3c). Sequencing reactions will start when dye-labeled sequencing substrates are introduced into a single molecule chamber, especially via laminar flow, and they dock onto the immobilized ClpXP proteins via diffusion within the chamber.

Consequent FRET time trajectories can be obtained at high time resolution (up to 10 milliseconds) with a state-of-the-art CCD camera (such as e.g. Andor, iXon, electron-multiplying CCD). For the best signal-to-noise ratio, a trio of Cyanine dyes (Cy3, Cy5 and Cy7) (Lee, J., Lee, S., Ragunathan, K., Joo, C., Ha, T., and Hohng, S. (2010). Single-molecule four-color FRET, Angew. Chem Int. Ed. Engl. 49, 9922-9925) can be used as FRET pairs. For minimal photo blinking and slow photo bleaching, an oxygen scavenger system (glucose oxidase and catalase) and a triplet-state quencher (Trolox) can be used. As it is preferred to prevent non-specific adsorption of proteins onto a surface, a quartz surface will be coated with polymer (PEG, polyethylene glycol). However, also other surfaces may be applied. For instance, bovine serum albumin or casein-coated surfaces may be applied. In case where instead of an optical signal, an electrical signal is evaluated (as in the case of a nanopore system), the surface may also be coated with proteins or lipid bilayers.

ClpX translocation should be fast enough that sequencing is completed before donor molecules photo bleach. A recent single-molecule study suggests that the speed of ClpX is 60 amino acids per second. This rate is converted into 6.7 seconds per sequencing (of a protein substrate of average size, ∼400 amino acids). This time window is well within the time scale of Cy3 observation, typically a few minutes when using a 0.1 sec time resolution. On the other hand, ClpX translocation should be slow enough for adequate photon statistics and reliable analysis of time traces. When we need to slow down the process, we need only to drop the ATP concentration (from millimolar to micromolar) because the translocation of ClpX is energy (ATP) dependent.

Hence, in an embodiment the functionalized protein is translocated with the immobilized ATP dependent protease based molecular transporter machine with a translocation speed through the detection area of the detector, wherein the translocation speed is selected from the range of 0.1-60 amino acids per second. Especially, the translocation speed is controlled by controlling an ATP concentration in the liquid.

Hence, in an embodiment, with an immobilized (ATP dependent protease based) molecular transporter machine the functionalized protein (in the liquid phase) is guided through a detection area of a detector.

Due to the low association affinity of ClpX monomers, it is desired to use an artificially linked hexameric form of ClpX for a single-molecule study. We have expressed and have purified this hexameric form of ClpX. ClpP, on the other hand, has a high association affinity, and so we have expressed ClpP as monomers.

ClpX and ClpP may form either a 1:2 or 2:2 (ClpX:ClpP) complex. For protein sequencing, we may especially use the 1:2 complex, in order to avoid processing of multiple substrates simultaneously by the same complex. This stoichiometry can be reliably attained using a super stoichiometric ratio, such as at least 3:1 ratio between ClpP tetradecamer and ClpX hexamer concentrations in mixing.

Unfolded proteins tend to aggregate. To prevent this from occurring with sequencing substrates, unfolded labeled substrates may be kept in a denaturant. Note that this denaturant may not interfere with the sequencing reaction because unfolded substrates will be introduced into a sequencing chamber at ∼1 nM concentration via dilution into a physiological buffer, and this will result in the low concentration of denaturant. To further minimize any aggregation, we can rapidly dilute sequencing substrates immediately prior to a sequencing reaction, following a procedure optimized by Meyer (Meyer, A.S., Gillespie, J.R., Walther, D., Millet, I.S., Doniach, S., and Frydman, J. (2003). Closing the folding chamber of the eukaryotic chaperonin requires the transition state of ATP hydrolysis. Cell 113, 369-381.).

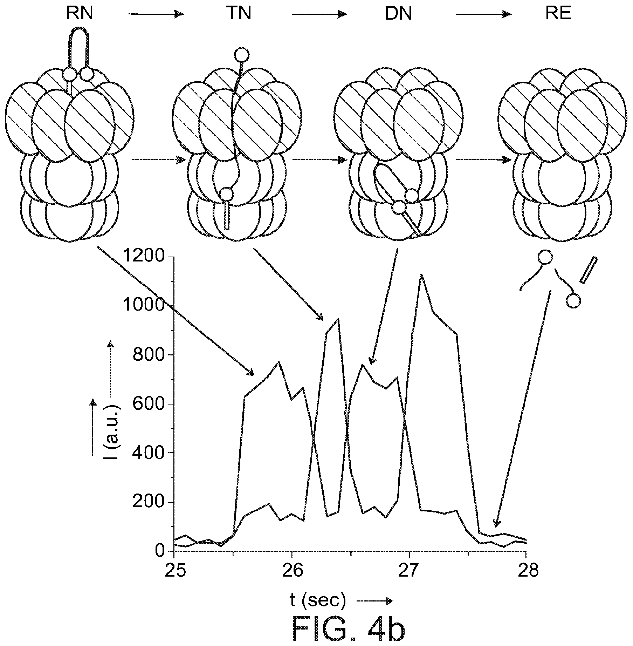

We hereby provide a demonstration of the ClpXP-based scanning process using the single-molecule FRET technique (Figure 4b). We generated an artificially linked hexameric form of ClpX and biotinylated its C-terminal end. We immobilized this nanochannel protein, in complex with the protease ClpP, on a quartz surface using streptavidin-biotin conjugation. In this sample chamber, we added peptides that contained a K and a C residue. The K residue was labeled with a Cy3 fluorophore containing an NHS ester group (green sphere in the figure), and the C residue was labeled with a Cy5 fluorophore containing a mono-maleimide group (red sphere). The peptide contained the ssrA-tag that is recognized by ClpX with high specificity.

We imaged the translocation process of the peptide through the nanochannel of ClpX using a lab-built total-internal-reflection microscope and recorded fluorescence signals using an electron-multiplying CCD camera. A sudden increase in the fluorescence signal (time at 25.5 sec in Figure Y) reports on the docking of a peptide to an immobilized ClpXP scanner. FRET between Cy3 and Cy5 is initially efficient due to the folded structure of the designed peptide, as shown by the high acceptor fluorescence (the donor time trace is a green line, and acceptor in red). When the peptide is pulled by ClpX and is translocated through the nanochannel of the ClpX via ATP hydrolysis, it becomes linearly stretched, the distance between Cy3 and Cy5 becomes greater, and the FRET efficiency becomes lower (time at 26.2 sec). This translocation process is followed by refolding of the peptide within the ClpP chamber (time at 26.5 sec) and its cleavage and subsequent dissociation events (time at 27.0 and 27.6 sec).

Hence, as described above, the invention provides in an embodiment a method for analysing a protein in a liquid comprising the protein, especially for determining the type of a protein in a liquid comprising the protein, the method comprising (a) functionalizing a protein with 2-4 types of amino acid labels, which are selective for 2-4 types of predefined protein amino acids, especially only two amino acid labels selective for only two amino acids, especially the C and K amino acids, (b) guiding in the liquid phase the functionalized protein with an immobilized ATP dependent protease based molecular transporter machine through a detection area of a detector, configured to detect a signal as function of the labels of the labelled amino acids; and (c) determining from the detected signal a sequence of the predefined amino acids. Especially, this analysis method further involves (d) comparing the sequence of the predefined amino acids with the occurrence of such sequence in a database of proteins and determining the type of protein in the liquid. In this way, the type of protein may be determined. Such database may in an embodiment be a remote database. For instance, the found sequence can be compared with data from the internet on known sequenced amino acids.

Especially, the method is an ex vivo method. The liquid mentioned above may be a body liquid, but may also be a diluted body liquid. Further, also other liquids are conceivable, such as cell extracts and organelles from bacteria, archaea, eukaryotes. Especially, the liquid is an aqueous liquid.

As also indicated above, the invention also provides a device for determining the type of protein in a liquid, the device comprising (a) an immobilized ATP dependent protease based molecular transporter machine configured to guide a protein that is functionalized with labels through a detection area of a detector, (b) said detector, configured to detect a signal as function of the labels of the labelled amino acids, (c) a processor unit, configured to identify from the detector signal a sequence of amino acids of the functionalized protein, and optionally further configured to compare the identified sequence of amino acids with the occurrence of such sequence in a database of proteins and to identify the type of protein.

Especially, the processor unit may be configured to compare the sequence of the predefined protein amino acids of the protein with the occurrence of such sequence in a database of proteins and determine the type of protein in the liquid, wherien the protein is functionalized with 2-8, such as especially only 2-4 types of amino acid labels, which are selective for 2-8, especially 2-4 types of predefined protein amino acids. Hence, the processor unit may be configured to determine based on only 2-8, especially 2-4 types of labeled amino acids, i.e. 2-8, especially 2-4 types of different amino acid selective labels, the type of protein when the respective protein amino acids (or at least part of the total number thereof) are labeled with these selective labels. Even only 2 types of labels, e.g. for lysine and cysteine, may be enough.

The label, for use in the method, may be used for fluorescence based analysis methods or the label may be used for an analysis method based on electronical signals (see below when describing in more detail the nanopore method). In principle, the fluorescent acceptors as described above, may also be applied in the nanopore method, though also other labels may be applied in the latter method, such as gold beads, quantum dots, and other solid-state nanoparticles. Hence, in an embodiment, the labels comprise fluorescent acceptors, wherein a fluorescent donor, configured to temporarily form a donor acceptor pair with one of the fluorescent acceptors, is configured within the detection area, and wherein the detector comprises a fluorescence microscope including a total-internal-reflection fluorescence (TIRF) microscope, a confocal fluorescence microscope, or a zero-mode waveguide-based fluorescence microscope. Hence, especially the detector comprises a fluorescence microscope including a total-internal-reflection fluorescence (TIRF) microscope, a confocal fluorescence microscope, or a zero-mode waveguide-based fluorescence microscope. In a specific embodiment, immobilized ATP dependent protease based molecular transporter machine comprises a fluorescent donor attached thereto. Even more especially, see also above, the molecular transporter machine is a ClpXP based molecular transporter machine, wherein the ClpXP based molecular transporter machine comprises an asymmetric ClpP chamber of ClpP monomers and at least one mutant ClpP monomer, in which the at least one mutant ClpP monomer cannot interact with ClpX, and wherien this at least one mutant ClpP is fluorescent donor labelled, or wherein the ClpXP based molecular transporter machine comprises of a ClpX protein comprising one fluorescent donor label per hexamer. As mentioned above, labels may be applied e.g. selected from one or more of the Cyanine family, the Alexa family, the Atto family, the Dy family, and the Rhodamine family, etc..

However, in yet another embodiment, the method comprises guiding the functionalized protein with the molecular transporter machine through a nanopore of a nanopore comprising filter having a nano-transporter side and an opposite side, wherein the detector comprises a detector unit configured to measure an electrical parameter between the nano-transporter side and an the opposite side of the nano-pore comprising filter, and wherein the electrical parameters is selected from the group consisting of a potential difference, a current and resistance. Hence, in a further embodiment, the device as described herein may further comprise a nanopore comprising filter having a nano-transporter side and an opposite side and wherein the detector is configured to measure an electrical parameter between the nano-transporter side and an the opposite side of the nano-pore comprising filter, wherein the electrical parameters is selected from the group consisting of a potential difference, a current and resistance, wherein the device is further configured to guide the protein that is functionalized with labels through the nanopore during use of the device. The pores of the nanopores may have diameters in the range of 0.1-10 nm. The width of the filter, i.e. the channel length of the nanopore may be in the range of 0.3-10 nm.

Instead of or in addition to determining the order of the (labeled) amino acids, the time interval between the (labeled) amino acids may (also) be determined. From this information (and the sequencing speed of the molecular transporter machine), the distance between adjacent (labeled) amino acids may be determined. This information can (further) be used to determine the type of protein (in the liquid). Hence, from the sensor signal, also a distance parameter between adjacent labeled AA can be determined. This distance parameter can also be applied to further determine the type of protein (in the liquid).

Herein, especially a molecular transport system for translocating a sequencing substrate with nanometer accuracy and also for slowing down the whole translocation process is applied. The fluorescence and nanopore community have been looking forward to a single-molecule protein sequencing approach for decades. Despite all the existing literature on molecular transport systems and the development of new single-molecule techniques, none from the community has come up with any similar design as ours. For instance, any dye-labeling of ClpXP or other transporters, is not known or suggested in the prior art.

The term "substantially" herein, such as in "substantially all emission" or in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices or apparatus herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments within the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The invention further applies to an apparatus or device comprising one or more of the characterizing features described in the description and/or shown in the attached drawings. The invention further pertains to a method or process comprising one or more of the characterising features described in the description and/or shown in the attached drawings.

The various aspects discussed in this patent can be combined in order to provide additional advantages. Furthermore, some of the features can form the basis for one or more divisional applications.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs.1a-1b depict some aspects of a principle of the invention;
Figs. 2a-2b schematically depict some aspects of an embodiment of the ATP dependent protease based molecular transporter, here being a ClpXP;
Figs. 3a-3c schematically depict some embodiments and variants on the method and device of the invention; and
Figs. 4a and 4b show some simulations and experimentation results.

The drawings are not necessarily on scale.

### DETAILED DESCRIPTION OF THE EMBODIMENTS

Fig. 1a schematically depicts a protein, such as an enzyme. The protein is indicated with reference 100. The protein 100 essentially consists of a chain of amino acids 110. Amino acids 110 that are labeled, are indicated with references 111. Reference L1 indicates a first label and reference L2 refers to a second label (i.e. amino acids with label L1 and L2, respectively). As indicated above, especially two amino acids may be labeled, such as lysine (about 1 out of 20 amino acids) and cysteine (about 1 out of 40 amino acids). As indicated above, a protein can be identified from the order of e.g. C (Cys) and K (Lys) residues only.

Fig. 1b shows a calculation on the prediction power of the suggested method. The prediction fidelity is proportional to the number of C and K residues in a sequencing substrate. The fingerprinting becomes reliable when the number is larger than 15, such as least 16, like especially at least 17. Over 25, the prediction power is 100%. Note that the number of at least 15 relates to the number of different amino acids labeled with different labels. Hence, 6 labeled C amino acids and 12 labeled K amino acids make 18 labeled amino acids, which will give a high predictive power. Hence, after measure at least in the order of 15-25 labeled amino acids, the determination can be conclusive.

Fig. 2a schematically depicts the ClpXP enzyme 200, with ClpX (ATPase), being indicated with reference 210 and with ClpP (protease) being indicated with reference 220. The individual monomers are indicated with references 211 (ClpX) and 221 (ClpP), respectively. Fig. 2b shows a cross-sectional view of ClpXP. A Cy5 and Cy7-labeled sequencing substrate 100, i.e. protein 100 (which has labeled amino acids, here again labeled with labels L1 and L2), is pulled down into the narrow channel 212 of ClpX 210. The stretched substrate is handed over to ClpP 220 where it is digested into a small fragment (∼7 amino acids). There will be FRET between Cy3 (donor, indicated with reference 50) near the ClpP exit port and Cy5 and Cy7 on a fragment within the ClpP chamber (see below).

The height of ClpX 210 is indicated with reference H1 (about 7,5 nm); the width is indicated with reference d2 (about 15 nm). The channel diameter of channel 212 is indicated with d1 (about 1 nm). The height of ClpP 220 is indicated with H2 (about 9 nm); the width (not indicated) is also about 9 nm; the width of chamber 223 is indicated with diameter d3 (which is about 5 nm).

Fig. 3a on the left shows an embodiment of immobilization of the ClpXP enzyme 200 at a surface 300, such as quartz. The immobilization is e.g. conducted by tagging ClpP proteins (enzymes) 200 with biotin 302 and introducing them onto a streptavidin-layered surface, such as quartz. Streptavidin is indicated with reference 303. The surface of the substrate can be precoated with a polymer, indicated with reference 301, such as PEG (polyethylene glycol). On the left hand side of fig. 3a, schematically the process is indicated (with the arrow). PEG may prevent non-specific adsorption of proteins (ClpXP and seqeuencing substrates) to a surface. Without PEG, ClpXP may lose its function, and the signals from seqeuencing substrates may non-specifically appear on a CCD screen. PEG may also provide biotin which Streptavidin binds to.

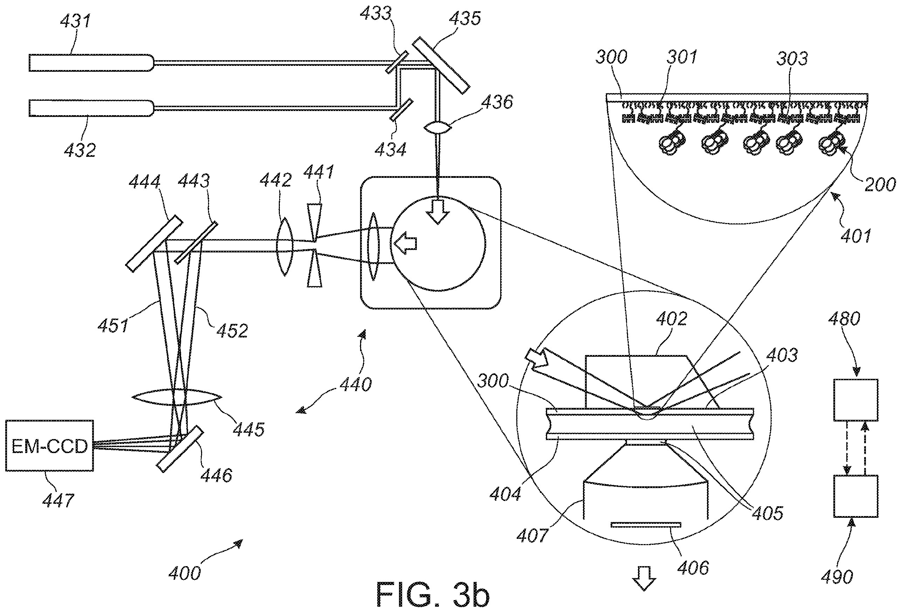

Fig. 3b schematically depicts a FRET based (detection) device for use in e.g. the method of the invention. The device is indicated with reference 400. References 431 and 432 indicated lasers, respectively, such as a 532 nm and 633 nm laser, respectively. Reference 434 is a mirror, reference 433 is a transmissive mirror (such as a dichroic mirror). Reference 433 may be composed of more than one mirror. Reference 435 is a mirror, and reference 436 is a lens, with e.g. a focal distance of 100 mm. reference 440 indicates a TIRF (total-internal-reflection fluorescence) microscope (see enlargement). Reference 441 indicates a slit and reference 442 indicates a lens, with e.g. a focal distance of 100 mm. reference 443 indicates a dichroic mirror (especially for the donor beam), and reference 444 indicates a mirror (especially for the acceptor beam). Reference 445 is again a lens, with focal distance of e.g. 150 mm; reference 446 indicates a mirror, and reference 447 indicates a detector, such as an EM-CCD (electron multiplying CCD). Reference 402 indicates a Pellin-Broca prism and reference 300 indicates the surface, here a quartz plate. On the surface 300, with respect to fig. 3a described immobilized ClpXP 200 is present. The evanescent field is indicated with reference 401. Reference 404 indicates a transparent cover, such as a glass cover slip. Reference 405 indicates a transparent medium, such as water. Reference 406 indicates a band pass filter and reference 407 indicates an objective lens (such as 60x water, NA 1.2). Reference 480 indicates a processor and reference 490 indicates an optional library (which may be remote, e.g. internet database with amino acid sequences of proteins).

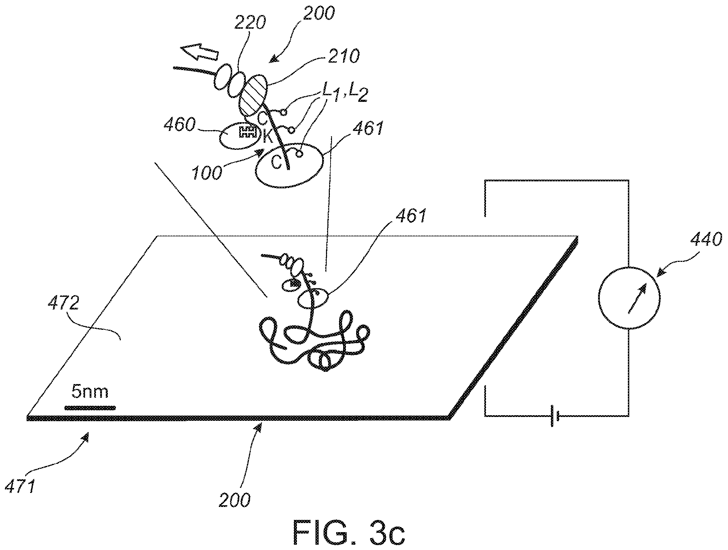

Fig. 3c schematically depicts an alternative embodiment of the device 1, with a plate 472 with a nanopore 461. The plate may e.g. of the following materials, silicon nitride ("SiN"), Si/SiO₂, Al₂O₃, polymer, graphene, BN An electric signal is measured between a nano-transporter side 472 and an opposite side 471. Here the detector 440 comprises a detector unit configured to measure an electrical parameter between the nano-transporter side and an the opposite side of the nanopore comprising filter. The electrical parameters is selected from the group consisting of a potential difference, a current and resistance. When the protein 200 passes through the nanopore 461 (from the opposite side to the nano-transporter side 462), due to the presence of the labels, the electrical signal will change. The signal change will depend upon the type of label. Note that in this case the labels are not necessarily luminescent. Further, note that the moecular transporter machine is configured to draw the protein 100 through the nanopore 461.

For example, our analysis (Figure 4a) shows that the predication fidelity (PF) does not drop significantly even when there are a number of swapping errors (NSE) present (i.e. the order of C and K is swapped during scanning) given a 40-long CK sequence. We performed this error analysis using both a point-point matching algorithm and the Smith-Waterman algorithm.

We hereby provide a demonstration of the ClpXP-based scanning process using the single-molecule FRET technique (Figure 4b). We generated an artificially linked hexameric form of ClpX and biotinylated its C-terminal end. We immobilized this nanochannel protein, in complex with the protease ClpP, on a quartz surface using streptavidin-biotin conjugation. In this sample chamber, we added peptides that contained a K and a C residue. The K residue was labeled with a Cy3 fluorophore containing an NHS ester group (green sphere in the figure), and the C residue was labeled with a Cy5 fluorophore containing a mono-maleimide group (red sphere). The peptide contained the ssrA-tag that is recognized by ClpX with high specificity. Reference RN, TN, DN, and RE indicate recognition, translocation, degradation, and release, respectively. In the graph, I on the y-axis indicates intensity (in arbitrary units), and t on the x-axis is time (in seconds).

## Claims

1. A method for determining the type of a protein in a liquid comprising the protein, the method comprising:
a) functionalizing a protein with at least 2 types of amino acid labels, which are selective for 2 types of predefined protein amino acids,
b) guiding in the liquid phase the functionalized protein with an immobilized ATP dependant protease based molecular transporter machine through a detection area of a detector, configured to detect a signal as function of the labels of the labelled amino acids;
c) determining from the detected signal a sequence of the predefined protein amino acids;
d) comparing the sequence of the predefined protein amino acids with the occurrence of such sequence in a database of proteins and determining the type of protein in the liquid.

2. The method according to claim 1, wherein the molecular transporter machine is a molecular transporter machine selected from the group of ATP dependent proteases consisting of a ClpXP, a ClpAP, a ClpCP, a ClpEP, a ClpYQ, a ClpB, a Lon, an FtsH, an archeal PAN, and a proteasome based molecular transporter machine, especially wherein the molecular transporter machine is a ClpXP based molecular transporter machine.

3. The method according to any one of the preceding claims, wherein only cysteine and lysine are labeled, and wherein the molecular transporter machine comprises a donor configured to probe an amino acid label.

4. The method according to any one of the preceding claims, wherein the labels comprise fluorescent acceptors, wherein a fluorescent donor, configured to temporarily form a donor acceptor pair with one of the fluorescent acceptors, is configured within the detection area, and wherein the detector comprises a fluorescence microscope including a total-internal-reflection fluorescence (TIRF) microscope, a confocal fluorescence microscope, or a zero-mode waveguide-based fluorescence microscope; and wherein the labels comprise an organic fluorophore selected from one or more of the Cyanine family, the Alexa family, the Atto family, the Dy family, and the Rhodamine family.

5. The method according to claim 4, wherein the molecular transporter machine is a ClpXP based molecular transporter machine, wherein (i) especially the ClpXP based molecular transporter machine comprises an asymmetric ClpP chamber of ClpP monomers and at least one mutant ClpP monomer, in which at least one mutant ClpP monomer cannot dock to ClpX, and wherein this at least one mutant ClpP is fluorescent donor labelled, or wherein (ii) especially. the ClpXP based molecular transporter machine comprises a fluorescent donor labelled ClpX in complex with an unlabeled ClpP protein.

6. The method according to any one of claims 1-5, wherein the method comprises guiding the functionalized protein with the molecular transporter machine through a nanopore comprising filter having a nano-transporter side and an opposite side, wherein the detector comprises a detector unit configured to measure an electrical parameter between the nano-transporter side and an the opposite side of the nano-pore comprising filter, and wherein the electrical parameter is selected from the group consisting of a potential difference, a current and resistance.

7. The method according to any one of the preceding claims, further comprising taging the protein with a tag that is recognizable by the molecular transporter machine.

8. The method according to any one of the preceding claims, wherein the functionalized protein is translocated with the immobilized ATP dependent protease based molecular transporter machine with a translocation speed through the detection area of the detector, wherein the translocation speed is selected from the range of 0.1-60 amino acids per second, especially wherein the translocation speed is controlled by controlling an ATP concentration in the liquid.

9. The method according to any one of the preceding claims, comprising detecting the presence of at least 15, especially at least 17, of the predefined protein amino acids in the protein to be identified, comprising functionalizing the protein with 2-4 types of amino acid labels, which are selective for 2-4 types of predefined protein amino acids, and, wherein the database is a remote database.

10. A device for determining the type of protein in a liquid, the device comprising:
a) an immobilized ATP dependent protease based molecular transporter machine configured to guide a protein that is functionalized with amino acid labels, which are selective for at least 2 types of predefined protein amino acids, through a detection area of a detector,
b) said detector, configured to detect a signal as function of the labels of the labelled amino acids,
c) a processor unit, configured to identify from the detector signal a sequence of amino acids of the functionalized protein, wherein the processor unit is further configured to compare the identified sequence of amino acids with the occurrence of such sequence in a database of proteins and to identify the type of protein.

11. The device according to claim 10, wherein the molecular transporter machine is a molecular transporter machine selected from the group of ATP dependent proteases consisting of i.a. a ClpXP, a ClpAP, a ClpCP, a ClpEP, a ClpYQ, a ClpB, a Lon, an FtsH, an archeal PAN and a proteasome based molecular transporter machine, especially wherein the molecular transporter machine is a ClpXP based molecular transporter machine.

12. The device according to any one of claims 10-11, wherein the detector comprises a fluorescence microscope including a total-internal-reflection fluorescence (TIRF) microscope, a confocal fluorescence microscope, or a zero-mode waveguide-based fluorescence microscope, and wherein especially the immobilized ATP dependent protease based molecular transporter machine comprises a fluorescent donor attached thereto.

13. The device according to any one of claims 10-12, wherein the molecular transporter machine is a ClpXP based molecular transporter machine, wherein the ClpXP based molecular transporter machine comprises an asymmetric ClpP chamber of ClpP monomers and at least one mutant ClpP monomer, in which the at least one mutant ClpP monomer cannot dock to ClpX, and wherien this at least one mutant ClpP is fluorescent donor labelled, especially wherein the molecular transporter machine is a ClpXP based molecular transporter machine, wherein the ClpXP based molecular transporter machine comprises a fluorescent donor labelled ClpX in complex with a ClpP protein.

14. The device according to any one of claims 10-13, wherein the device further comprises a nanopore comprising filter having a nano-transporter side and an opposite side and wherein the detector is configured to measure an electrical parameter between the nano-transporter side and an the opposite side of the nano-pore comprising filter, wherein the electrical parameters is selected from the group consisting of a potential difference, a current and resistance, wherein the device is further configured to guide the protein that is functionalized with labels through the nanopore during use of the device.

15. The device according to any one of claims 10-14, wherein an immobilized ATP dependent protease based molecular transporter machine is configured to guide a protein that is functionalized with amino acid labels, which are selective for 2-4 types of predefined protein amino acids, through a detection area of a detector, wherein the detector is configured to compare the sequence of the predefined protein amino acids of the protein with the occurrence of such sequence in a database of proteins and determining the type of protein in the liquid, wherien the protein is functionalized with only 2-4 types of amino acid labels, which are selective for 2-4 types of predefined protein amino acids.

## Patentansprüche

1. Verfahren zum Bestimmen des Typs eines Proteins in einer Flüssigkeit, die Protein umfasst,
wobei das Verfahren Folgendes umfasst:
a) das Funktionalisieren eines Proteins mit mindestens 2 Typen Aminosäurelabeln, die für 2 Typen vordefinierter Proteinaminosäuren selektiv sind,
b) das Führen, in der flüssigen Phase, des funktionalisierten Proteins mit einer immobilisierten, molekularen Transportermaschine auf der Basis von von ATP abhängiger Protease durch einen Detektionsbereich eines Detektors, der konfiguriert ist, um ein Signal in Abhängigkeit von den Labeln der gelabelten Aminosäuren zu detektieren;
c) das Bestimmen, aus dem detektieren Signal, einer Sequenz der vordefinierten Proteinaminosäuren;
d) das Vergleichen der Sequenz der vordefinierten Proteinaminosäuren mit dem Vorhandensein einer derartigen Sequenz in einer Datenbase von Proteinen und das Bestimmen des Typs Protein in der Flüssigkeit.

2. Verfahren nach Anspruch 1, wobei die molekulare Transportermaschine eine molekulare Transportermaschine ist ausgewählt aus der Gruppe von von ATP abhängigen Proteasen bestehend aus einem ClpXP, einem ClpAP, einem ClpCP, einem ClpEP, einem ClpYQ, einem ClpB, einem Lon, einem FtsH, einem archealischen PAN, und einer auf Proteasom basierenden molekularen Transportermaschine, wobei insbesondere die molekulare Transportermaschine eine molekulare Transportermaschine auf der Basis von ClpXP ist.

3. Verfahren nach irgendeinem der vorhergehenden Ansprüche, wobei nur Cystein und Lysin gelabelt sind und wobei die molekulare Transportermaschine einen Donor umfasst, der zum Sondieren eines Aminosäurelabels konfiguriert ist.

4. Verfahren nach irgendeinem der vorhergehenden Ansprüche, wobei die Label fluoreszierende Akzeptoren umfassen, wobei ein fluoreszierender Donor, der zum zeitweiligen Bilden eines Donor-Akzeptor-Paars mit einem der fluoreszierenden Akzeptoren konfiguriert ist, innerhalb des Detektionsbereichs konfiguriert ist, und wobei der Detektor ein Fluoreszenzmikroskop umfasst, das ein total internes Reflexionsfluoreszenz-(TIRF) Mikroskop, ein konfokales Fluoreszenzmikroskop oder ein
Nullmodus-Fluoreszenzmikroskope auf Wellenleiterbasis umfasst; und wobei die Label ein organisches Fluorophor umfassen ausgewählt aus einem oder mehreren der Cyanin-Familie, der Alexa-Familie, der Atto-Familie, der Dy-Familie und der Rhodamin-Familie.

5. Verfahren nach Anspruch 4, wobei die molekulare Transportermaschine eine molekulare Transportermaschine auf der Basis von ClpXP ist, wobei (i) insbesondere die molekulare Transportermaschine auf der Basis von ClpXP eine asymmetrische ClpP-Kammer von ClpP-Monomeren und mindestens ein mutantes ClpP-Monomer umfasst, wobei mindestens ein mutantes ClpP-Monomer nicht an ClpX anlegen kann und wobei dieses mindestens eine mutante ClpP mit fluoreszierendem Donor gelabelt ist oder wobei (ii) insbesondere die molekulare Transportermaschine auf der Basis von ClpXP ein mit fluoreszierendem Donor gelabeltes ClpX in einem Komplex mit einem ungelabelten ClpP-Protein umfasst.

6. Verfahren nach irgendeinem der Ansprüche 1 bis 5, wobei das Verfahren das Führen des funktionalisierten Proteins mit der molekularen Transportermaschine durch eine Nanopore umfasst, die ein Filter umfasst, das eine Nanotransporterseite und eine entgegengesetzte Seite aufweist, wobei der Detektor eine Detektoreinheit umfasst, die konfiguriert ist, um einen elektrischen Parameter zwischen der Nanotransporterseite und einer entgegengesetzten Seite der Nanopore, die das Filter umfasst, zu messen, und wobei der elektrische Parameter aus der Gruppe ausgewählt wird bestehend aus einem Potentialunterschied, einem Strom und Widerstand.

7. Verfahren nach irgendeinem der vorhergehenden Ansprüche, ferner das Markieren des Proteins mit einem Marker umfassend, der durch die molekulare Transportermaschine erkennbar ist.

8. Verfahren nach irgendeinem der vorhergehenden Ansprüche, wobei das funktionalisierte Protein mit der immobilisierten, molekularen Transportermaschine auf der Basis von von ATP abhängiger Protease mit einer Translokationsgeschwindigkeit durch den Detektionsbereich des Detektors transloziert wird, wobei die Translokationsgeschwindigkeit aus dem Bereich von 0,1- 60 Aminosäuren pro Sekunde, ausgewählt wird, wobei insbesondere die Translokationsgeschwindigkeit durch Regulieren einer ATP-Konzentration in der Flüssigkeit reguliert wird.

9. Verfahren nach irgendeinem der vorhergehenden Ansprüche, umfassend das Detektieren des Vorliegens von mindestens 15, insbesondere mindestens 17, der vordefinierten Proteinaminosäuren in dem zu identifizierenden Protein, umfassend das Funktionalisieren des Proteins mit 2 - 4 Typen Aminosäurelabeln, die für 2 - 4 Typen vordefinierter Proteinaminosäuren selektiv sind und wobei die Datenbase eine entfernte Datenbase ist.

10. Vorrichtung zum Bestimmen des Typs von Protein in einer Flüssigkeit, wobei die Vorrichtung Folgendes umfasst:
a) eine immobilisiert, molekulare Transportermaschine auf der Basis von von ATP abhängiger Protease, die zum Führen eines Proteins, das mit Aminosäurelabeln funktionalisiert ist, die für mindestens 2 Typen vordefinierter Proteinaminosäuren selektiv sind, durch einen Detektionsbereich eines Detektors konfiguriert ist,
b) wobei der Detektor zum Detektieren eines Signals in Abhängigkeit von den Labeln der gelabelten Aminosäuren konfiguriert ist,
c) eine Prozessoreinheit, die zum Identifizieren, aus dem Detektorsignal, einer Sequenz von Aminosäuren des funktionalisierten Proteins konfiguriert ist, wobei die Prozessoreinheit ferner zum Vergleichen der identifizierten Sequenz von Aminosäuren mit dem Vorhandensein einer derartigen Sequenz in einer Datenbase von Proteinen und zum Identifizieren des Typs Protein konfiguriert ist.

11. Vorrichtung nach Anspruch 10, wobei die molekulare Transportermaschine eine molekulare Transportermaschine ist ausgewählt aus der Gruppe von von ATP abhängigen Proteasen bestehend aus, unter anderem, einem ClpXP, einem ClpAP, einem ClpCP, einem ClpEP, einem ClpYQ, einem ClpB, einem Lon, einem FtsH, einem archealischen PAN und einer auf Proteasom basierenden molekularen Transportermaschine, wobei insbesondere die molekulare Transportermaschine eine molekulare Transportermaschine auf der Basis von ClpXP ist.

12. Vorrichtung nach irgendeinem der Ansprüche 10 - 11, wobei der Detektor ein Fluoreszenzmikroskop umfasst, das ein total internes Reflexionsfluoreszenz-(TIRF) Mikroskop p, ein konfokales Fluoreszenzmikroskop oder ein Nullmodus-Fluoreszenzmikroskop auf Wellenleiterbasis umfasst; und wobei insbesondere die immobilisierte, molekulare Transportermaschine auf der Basis von von ATP abhängiger Protease einen daran angebrachten fluoreszierenden Donor umfasst.

13. Vorrichtung nach irgend einem der Ansprüche 10 - 12, wobei die molekulare Transportermaschine eine molekulare Transportermaschine auf der Basis von ClpXP ist, wobei die molekulare Transportermaschine auf der Basis von ClpXP eine asymmetrische großes ClpP-Kammer von ClpP-Monomeren und mindestens ein mutantes ClpP-Monomer umfasst, wobei das mindestens eine mutante ClpP-Monomer nicht an ClpX anlegen kann und wobei dieses mindestens eine mutante ClpP mit fluoreszierendem Donor gelabelt ist, wobei insbesondere die molekulare Transportermaschine auf der Basis von ClpXP ein mit fluoreszierendem Donor gelabeltes ClpX in einem Komplex mit einem ungelabelten ClpP-Protein umfasst.

14. Vorrichtung nach irgendeinem der Ansprüche 10 - 13, wobei die Vorrichtung ferner eine Nanopore umfasst, die ein Filter umfasst, das eine Nanotransporterseite und eine entgegengesetzte Seite aufweist und wobei der Detektor konfiguriert ist, um einen elektrischen Parameter zwischen der Nanotransporterseite und einer entgegengesetzten Seite der Nanopore, die das Filter umfasst, zu messen, wobei der elektrische Parameter aus der Gruppe ausgewählt wird bestehend aus einem Potentialunterschied, einem Strom und Widerstand, wobei die Vorrichtung ferner zum Führen des Proteins, das mit Labeln funktionalisiert ist, durch die Nanopore während der Verwendung der Vorrichtung konfiguriert ist.

15. Vorrichtung nach irgend einem der Ansprüche 10 - 14, wobei eine immobilisierte, molekulare Transportermaschine auf der Basis von von ATP abhängiger Protease konfiguriert ist, um ein Protein, das mit Aminosäurelabeln funktionalisiert ist, die für 2 - 4 Typen vordefinierter Proteinaminosäuren selektiv sind, durch einen Detektionsbereich eines Detektors zu führen, wobei der Detektor zum Vergleichen der Sequenz vordefinierter Proteinaminosäuren des Proteins mit dem Vorkommen einer derartigen Sequenz in einer Datenbase von Proteinen und zum Bestimmen des Typs von Protein in der Flüssigkeit konfiguriert ist, wobei das Protein mit nur 2 - 4 Typen Aminosäurelabeln funktionalisiert ist, die für 2 - 4 Typen vordefinierter Proteinaminosäuren selektiv sind.

## Revendications

1. Procédé pour déterminer le type d'une protéine dans un liquide comprenant la protéine, le procédé comprenant :
a) la fonctionnalisation d'une protéine avec au moins 2 types d'étiquettes d'acide aminé, qui sont sélectifs pour 2 types d'acides aminés protéiques prédéterminés,
b) le guidage dans la phase liquide de la protéine fonctionnalisée avec une machine de transport moléculaire basée sur une protéase immobilisée ATP dépendante à travers une zone de détection d'un détecteur, configuré pour détecter un signal comme une fonction des étiquettes des acides aminés étiquetés ;
c) la détermination à partir du signal détecté d'une séquence des acides aminés protéiques prédéfinis ;
d) la comparaison de la séquence des acides aminés protéiques prédéfinis avec la survenance d'une telle séquence dans une base de données de protéines et la détermination du type de protéine dans le liquide.

2. Procédé selon la revendication 1, dans lequel la machine de transport moléculaire est une machine de transport moléculaire sélectionnée à partir du groupe de protéases ATP dépendantes consistant en une ClpXP, une ClpAP, une ClpCP, une ClpEP, une ClpYQ, une ClpB, une Lon, une FtsH, une PAN archéale, et une machine de transport moléculaire basée sur un protéasome, particulièrement dans lequel la machine de transport moléculaire est une machine de transport moléculaire basée sur ClpXP.

3. Procédé selon l'une quelconque des revendications précédentes, dans lequel seulement la cystéine et la lysine sont étiquetées, et dans lequel la machine de transport moléculaire comprend un donneur configuré pour sonder une étiquette d'acide aminé.

4. Procédé selon l'une quelconque des revendications précédentes, dans lequel les étiquettes comprennent des accepteurs fluorescents, dans lequel un donneur fluorescent, configuré pour temporairement former un couple d'accepteurs donneurs avec un des accepteurs fluorescents, est configuré à l'intérieur de la zone de détection, et dans lequel le détecteur comprend un microscope à fluorescence incluant un microscope à fluorescence à réflexion interne totale (TIRF), un microscope à fluorescence à foyer commun, ou un microscope à fluorescence à base de guide d'ondes en mode zéro ; et dans lequel les étiquettes comprennent un fluorophore organique sélectionné à partir d'une ou plusieurs de la famille Cyanine, la famille Alexa, la famille Atto, la famille Dy, et la famille Rhodamine.

5. Procédé selon la revendication 4, dans lequel la machine de transport moléculaire est une machine de transport moléculaire basée sur ClpXP, dans lequel (i) particulièrement la machine de transport moléculaire basée sur ClpXP comprend une chambre ClpP asymétrique de monomères ClpP et au moins un monomère ClpP mutant, dans lequel au moins un monomère ClpP mutant ne peut pas s'amarrer à ClpX, et dans lequel cet au moins un mutant ClpP est un donneur fluorescent étiqueté, ou dans lequel (ii) particulièrement la machine de transport moléculaire basée sur ClpXP comprend un donneur fluorescent étiqueté ClpX en complexe avec une protéine ClpP non étiquetée.

6. Procédé selon l'une quelconque des revendications 1 à 5, dans lequel le procédé comprend le guidage de la protéine fonctionnalisée avec la machine de transport moléculaire à travers un nanopore comprenant un filtre ayant un côté nano-transporteur et un côté opposé, dans lequel le détecteur comprend une unité formant détecteur configurée pour mesurer un paramètre électrique entre le côté nano-transporteur et le côté opposé du nanopore comprenant le filtre, et dans lequel le paramètre électrique est sélectionné à partir du groupe constitué d'une différence de potentiel, d'un courant et d'une résistance.

7. Procédé selon l'une quelconque des revendications précédentes, comprenant en outre le marquage de la protéine avec un marqueur qui est reconnaissable par la machine de transport moléculaire.

8. Procédé selon l'une quelconque des revendications précédentes, dans lequel la protéine fonctionnalisée subit une translocation avec la machine de transport moléculaire basée sur une protéase immobilisée ATP dépendante à une certaine vitesse de translocation à travers la zone de détection du détecteur, dans lequel la vitesse de translocation est sélectionnée à partir de la plage de 0,1 à 60 acides aminés par seconde, particulièrement dans lequel la vitesse de translocation est commandée en commandant une concentration d'ATP dans le liquide.

9. Procédé selon l'une quelconque des revendications précédentes, comprenant la détection de la présence d'au moins 15, particulièrement au moins 17, des acides aminés protéiques prédéfinis dans la protéine à identifier, comprenant la fonctionnalisation de la protéine avec 2 à 4 types d'étiquette d'acide aminé, qui sont sélectifs pour 2 à 4 types d'acides aminés protéiques prédéfinis et, dans lequel la base de données est une base de données distante.

10. Dispositif pour déterminer le type de protéine dans un liquide, le dispositif comprenant :
a) une machine de transport moléculaire basée sur une protéase immobilisée ATP dépendante configurée pour guider une protéine qui est fonctionnalisée avec des étiquettes d'acide aminé, qui sont sélectives pour au moins 2 types d'acides aminés protéiques prédéfinis, à travers une zone de détection d'un détecteur,
b) ledit détecteur, configuré pour détecter un signal comme une fonction des étiquettes des acides aminés étiquetés,
c) une unité formant processeur, configurée pour identifier à partir du signal de détecteur une séquence d'acides aminés de la protéine fonctionnalisée, dans laquelle l'unité formant processeur est en outre configurée pour comparer la séquence identifiée d'acides aminés avec la survenance d'une telle séquence dans une base de données de protéines et pour identifier le type de protéine.

11. Dispositif selon la revendication 10, dans lequel la machine de transport moléculaire est une machine de transport moléculaire sélectionnée à partir du groupe de protéases ATP dépendantes consistant, entre autres, en une ClpXP, une ClpAP, une ClpCP, une ClpEP, une ClpYQ, une ClpB, une Lon, une FtsH, une PAN archéale et une machine de transport moléculaire basée sur un protéasome, particulièrement dans lequel la machine de transport moléculaire est une machine de transport moléculaire basée sur ClpXP.

12. Dispositif selon l'une quelconque des revendications précédentes 10 à 11, dans lequel le détecteur comprend un microscope à fluorescence incluant un microscope à fluorescence à réflexion interne totale (TIRF), un microscope à fluorescence à foyer commun, ou un microscope à fluorescence à base de guide d'ondes en mode zéro, et dans lequel particulièrement la machine de transport moléculaire basée sur une protéase immobilisée ATP dépendante comprend un donneur fluorescent attaché à celle-ci.

13. Dispositif selon l'une quelconque des revendications 10 à 12, dans lequel la machine de transport moléculaire est une machine de transport moléculaire basée sur ClpXP, dans laquelle la machine de transport moléculaire basée sur ClpXP comprend une chambre ClpP asymétrique de monomères ClpP et au moins un monomère ClpP mutant, dans lequel l'au moins un monomère ClpP mutant ne peut pas s'amarrer à ClpX, et dans lequel cet au moins un ClpP mutant est un donneur fluorescent étiqueté, particulièrement dans lequel la machine de transport moléculaire est une machine de transport moléculaire basée sur ClpXP, dans laquelle la machine de transport moléculaire basée sur ClpXP comprend un donneur fluorescent étiqueté ClpX en complexe avec une protéine ClpP.

14. Dispositif selon l'une quelconque des revendications 10 à 13, dans lequel le dispositif comprend en outre un nanopore comprenant un filtre ayant un côté nano-transporteur et un côté opposé et dans lequel le détecteur est configuré pour mesurer un paramètre électrique entre le côté nano-transporteur et le côté opposé du nanopore comprenant le filtre, dans lequel les paramètres électriques sont sélectionnés à partir du groupe consistant en une différence de potentiel, un courant et une résistance, dans lequel le dispositif est en outre configuré pour guider la protéine qui est fonctionnalisée avec des étiquettes à travers le nanopore pendant l'utilisation du dispositif.

15. Dispositif selon l'une quelconque des revendications 10 à 14, dans lequel une machine de transport moléculaire basée sur une protéase immobilisée ATP dépendante est configurée pour guider une protéine qui est fonctionnalisée avec des étiquettes d'acide aminé, qui sont sélectives pour 2 à 4 types d'acides aminés protéiques prédéfinis, à travers une zone de détection d'un détecteur, dans lequel le détecteur est configuré pour comparer la séquence des acides aminés protéiques prédéfinis de la protéine avec la survenance d'une telle séquence dans une base de données de protéines et déterminer le type de protéine dans le liquide, dans lequel la protéine est fonctionnalisée avec seulement 2 à 4 types d'étiquettes d'acide aminé, qui sont sélectifs pour 2 à 4 types d'acides aminés protéiques prédéfinis.
